# EUROPEAN PATENT APPLICATION

(11) **EP 3 961 238 A1**
(43) Date of publication of application: **02.03.2022**
(21) Application number: 21185537.4
(22) Date of filing: 14.07.2021
(51) Int. Cl.: G01R 33/54, A61B 5/055, G16H 40/60

(54) **SYSTEM AND METHOD FOR STANDARDIZED MRI EXAMINATIONS WITH PATIENT-CENTRIC SCAN WORKFLOW ADAPTATIONS**

(30) Priority: 27.08.2020 US 202063071057 P
(71) Applicant: Siemens Healthcare GmbH, 91052 Erlangen (DE)
(72) Inventor: Arroyo Camejo, Silvia Bettina, 90763 Fürth (DE); Gühring, Jens, 91052 Erlangen (DE); Huwer, Stefan, 91056 Erlangen (DE); Meyer, Heiko, 91080 Uttenreuth (DE); Schwarz, Raphael, 91088 Bubenreuth (DE); Wohlers, Julian, 91052 Erlangen (DE)

(57) **Abstract**

The invention describes a system (12) for standardized MRI examinations with patient-centric scan workflow (W) adaptations comprising:
- a protocol management system (21) for setup and/or to maintain scan programs (Pr) and/or scan protocols (P, P1) centrally,
- a scan queue designed to provide a representation of past and planned scan workflow steps,
- a scan workflow guidance system (22) which is configured to use a preconfigured scan protocol (P, P1) and/or scan program (Pr) by patient specific adaptions.

The invention further describes a related method, a scan workflow guidance system and a magnetic resonance imaging system.

## Description

The invention describes a system and a method for standardized MRI examinations with patient-centric scan workflow adaptations, especially for controlling a magnetic resonance imaging ("MRI") system.

Magnetic resonance imaging is one of the most powerful, diagnostic tools available. It allows for imaging of biological structures and tissues in a non-invasive fashion. MRI is fundamentally based on the targeted manipulation of the proton spins within the biological specimen by means of RF-fields and recording the spins' response as the current signal induced into receiver coils. Conventionally, a large, static magnetic field is required to prepare sufficient spin magnetization along the z-axis. Spatial encoding of the spin information is performed through time dependent gradients applied along x-, y-, and z-axis.

Besides it's non-invasive nature, the power of MRI originates from its versatility/configurability. Depending on the composition of the spin manipulating RF- and gradient pulse sequences, different tissue properties can be probed and visualized. In MRI a large number of so-called sequences (i.e., specific patterns of RF pulses and gradient pulses with certain timing constraints) have been developed, with the goal to, e.g., allow the visualization of particular properties of the imaged tissue or to adapt the image acquisition to specific requirements (e.g., an image acquisition insensitivity to patient motion or a very fast acquisition). Conventionally, the user of the magnetic resonance scanner ("MR scanner") is granted access to fine-tune a large set of the adjustable sequence parameters through the scan user interface (UI) in order to empower him/her to customize the imaging settings for the specific conditions and requirements of each patient. Both the individual scan protocols (i.e., each combination of sequence, reconstruction, and parameter set for each MR scan) and the entire scan program (i.e., the entire set of different protocols adding up to one patient's MR examination) may - dependent on the MR scanner user's experience level - be adapted to the specific patient's needs. This multitude of adjustable parameters in MRI - all influencing the image acquisition in one way or another - results in the handling of the MR scanner being a highly complex task. For this reason, MRI technicians require years of theoretical training followed by years of practical work experience before fully mastering this challenging imaging modality.

Today, healthcare providers are facing a number of challenging factors. In the area of diagnostic imaging, increasing patient volumes and lowering reimbursement rates increase the time pressure for each patient scan. Simultaneously, consolidation of healthcare providers requires consistent management of a larger fleet of diagnostic imaging scanners to ensure standardization of imaging protocols and consistent image quality across different scanner types and hospital/practice sites, independent of the experience level of the user. The latter is a particularly large challenge due to the complex, highly manual usage of the MR and the fact that training and experience levels within the MR scanner user workforce may vary dramatically across the entire operation of the healthcare provider. Another challenge is for the healthcare provider to establish a suitable balance between standardization and customization of the diagnostic imaging workflow: In large healthcare provider's organizations imaging protocols and programs need to be standardized, yet allowing for well-controlled degrees of customization of the image acquisition pipeline and overall scan workflow considering each patient's special needs and conditions, while ensuring optimal, diagnostic image quality - all of which is essential for precision medicine. In case customization is required to cater to the special needs of the patients it is difficult for the healthcare provider to ensure a standardized way of customization independently from the user who is conducting the customization.

The large spectrum of possible sequence and parameter configuration in MR imaging is conventionally reduced to a large, but finite set of imaging protocols (usually preconfigured by application specialists of the fabricator and expert personnel of the healthcare provider) and scan programs composed thereof, which the MR user can select in the scan UI during the preparatory phase of the MR examination. The scan programs are often sorted according to application/body region of interest (e.g., head, spine or knee) and most frequent indications/pathologies (e.g., screening, tumor or multiple sclerosis).

After selection of the appropriate preconfigured protocol (as prescribed by radiologist and using MR users experience) from the scan UI, the user starts the MR exam. During the MR exam the user conventionally needs to complete manual tasks at several points during the MR exam (e.g., slice planning, adapting protocol parameters). Oftentimes and dependent on the user's experience level, the user may perform modification to the preconfigured scan workflow (e.g., changing or adding of scan steps as required by the current patient).

Associated with this state-of-the-art approach to scan work-flow preparation and conduct the following problems occur:
1) Manually preconfigured protocols available at different MR scanners at the healthcare provider's site may vary. In combination with varying experience levels of the MR scanner user, this can lead to inconsistent usage of scan protocols for identical patients and patient indication dependent on the scanner type and MR user.
2) The varying experience levels across the MR user workforce in combination with the necessity to perform manual configuration during conduct of the MR scan protocols may result in unwarranted variations in image quality and overall scan workflow dependent on user's experience levels and notion.
3) Strictly rigid standardization of MR scan protocols (e.g., by scan indication) may lead to inflexibility to adapt to patient specific needs or to further disseminate incidental findings manifesting during the progress of the scan work-flow.

Currently, challenge 1) is addressed by manually configuring similar scan protocols and programs on each of the scanners within the healthcare provider's fleet. This is a tedious and inefficient process and it remains challenging to establish consistency across the scanner fleet. Recently, the Siemens Healthineers Dot Cockpit allows management and transfer of preconfigured scan protocols between the healthcare provider's Siemens scanner.

Challenge 2) is partially addressed by the healthcare providers setting operation-wide standards for scan protocols and programs that the MR scanner users are prescribed to follow. The healthcare providers are, however, not equipped with appropriate tools to consistently manage and maintain these standards and easily make them available to the MR scanner user. The MR protocol manager as part of the Siemens Healthineers MR Dot Cockpit represents a centralized management tool, allowing for an operation wide configuration of custom scan protocols and scan programs. This is an important first component of the overall solution, but selection of the appropriate scan program based on patient indication or/and suspected pathologies may remain challenging for the unexperienced MR scanner user.

Challenge 3) is currently addressed by the MR scanner user being instructed to use standardized, predefined scan programs (sometimes even password protected against modification). Depending on the healthcare provider's preferences either a) all or b) only highly qualified (admin) MR scanner users may be able to perform scan program modifications in order to accommodate patient-specific adaptations. Approach a) however, introduces frequent deviations from the standardized scan programs, which may depend primarily on the MR scanner user's judgement and experience level. Approach b) additionally suffers from the inability to adapt to patient-specific needs, when a non-admin MR scanner user is performing the scan examination. In all scenarios, a broad variability in MR scan workflow and final MR image sets is the result, while patient-specific modifications to the scan work-flow might be highly user dependent.

It is the object of the present invention to improve the known systems, devices, and methods to facilitate an improvement in controlling magnetic resonance imaging systems.

The strategic targets of this invention solving the challenge described in the above are especially:
1) Standardization of MR imaging protocols and programs across the healthcare provider's organization.
2) Automation of protocol adjustments during the scan work-flow as conventionally performed by the MR scanner user.
3) Smart, live adaptations of the preconfigured scan program (i.e., adaptations of scan parameters, addition or removal of scan protocols to the scan workflow) considering the individual patient characteristics (e.g., scan indication, health record, behavior during scan, findings in the initial MR images) enabling a "standardized customization".

This object is achieved by a method according to claim 1, a method according to claim 7, a scan workflow guidance system according to claim 10 and a magnetic resonance imaging system according to claim 13.

Central features required to implement this invention's strategy are especially:
a) Equipping the healthcare provider with a centralized system to setup and manage its MR scan protocols and scan programs in a standardized and consistent fashion across the entire fleet of scanners.
b) Enabling the MR scanner user to easily pull the right protocol for each patient's scan indication from the centrally managed protocol repository without the need for the MR user to complete manual tasks during protocol setup or scan acquisition that require detailed domain knowledge or a high experience level from the side of the MR user. Previously manual planning steps and new quality assurance steps are automatized through Smart Components.
c) The MR scanner analyzing initial scan indication data and MR image data as available during the scan workflow progresses and either recommending protocol adaptations and the addition/removal of scan steps to the MR user as required by the current patient or executing those scan workflow adaptations autonomously.

A system according to the invention for standardized MRI examinations with patient-centric scan workflow adaptations comprises the following components:
- Optionally: A scan-user-interface, which is configured to modify a scan workflow by a user,
- a protocol management system for setup and/or maintain scan programs and/or scan protocols centrally,
- a scan queue designed to provide a representation of past and planned scan workflow steps (the workflow steps comprising a number of protocols),
- a scan workflow guidance system which is configured to use a preconfigured scan protocol and/or scan program by patient specific adaptions (i.e. to suggest or execute patient specific adaptions), preferably wherein the scan protocols and/or scan programs, which are selected by a user, are getting adapted based on image data and/or user selected presetting.

The scan-user-interface (that may also be called "Scan UI" (UI = User Interface) serves to provide inputs of a user. It is an optional, yet preferred, feature that allows interactions and corrections by a user.

The Scan UI (especially displayed to the MR scanner user at scan acquisition time) ideally offers a minimal number of input options to the MR scanner user to directly or indirectly modify the scan workflow. It can be used to insert the scan indication or suspected pathologies of the patient.

In addition, the scan UI allows the user to add a set of standard protocols of the respective application (e.g., standard neurological exam), regardless of the recommendations determined by the scan workflow guidance system downstream.

The scan UI may be equipped with an option to review and edit scan work-flow adaptation as suggested by the scan workflow guidance system.

Although it could be advantageous that a user has a great variety of adjusting the system and/or the protocols, e.g., modify the scan workflow, insert the scan indication or suspected pathologies or add a set of standard protocols. For example, could the scan UI provide a user the possibility to input either the initial indication of the patient or at least one scan protocol which would be a modification of an (empty) scan workflow. However, it is not required in every possible case that the scanner user selects any protocol or an initial indication at all, since the system could start with a predefined protocol in every case or receive the scan indication (and thus the suitable scan program selection) electronically from a system, e.g., the RIS. Selecting a protocol or a patient indication (and optionally stating if a set of standard protocols should be used) has the advantage that a patient could be examined more effective. It should be noted that also an admin or master user, who (at configuration time, independent of any exam) could configure the available scan programs which are associated with a given indication.

The degree to which the scan program can be modified by the MR scanner user (after being loaded preconfigured from the protocol management system) may be controlled by a tiered privilege level concept, allowing deviations from the preconfigured standard (i.e., healthcare provider standards and live guidance through the scan workflow guidance system) only to the most senior MR scanner users in order to handle complex, non-standard MR examinations. The scan UI may contain further HMI elements. The user that is able to input and/or change protocols may be designated as "master-user".

It is particularly preferred that the scan UI is designed to fetch protocols from a central location (e.g., the protocol management system).

The protocol management system is made to host a number of scan protocols and/or scan programs and especially also scan workflows. It is preferred that scan protocols and scan programs can be setup and maintained centrally by the healthcare provider to establish and maintain an operation-wide scan program standard for each scan indication. A default set of scan protocols and scan programs may be provided by the scanner fabricator. Protocols and programs may be stored in a centralized, operation-wide accessible database (with or without tiered access control).

Possible realizations of this protocol database might range from a simple table with attribute columns, through a database with attribute and property tags to a more complex mapping of protocol attributes and properties or a decision tree structure.

At scan time, the protocol management system allows the MR scanner user to pull the most appropriate, preconfigured scan program for a large (or ideally a full) set of clinical MR scan indication on each scanner type of the healthcare provider's enterprise.

One preferred option of the scan protocol & program manager would include the capability to transfer scan protocols and programs from one scanner configuration to another (e.g., from a 3 T scanner to a 1.5 T scanner). One way to facilitate this scan protocol & program transfer in a consistent fashion is disclosed in the Siemens Healthineers patent US 20170123612 A1 Enterprise Protocol Management.

The system comprises a scan queue, wherein any memory device (of the system or another system (e.g., an MRI system) could be used as scan queue. The scan queue provides a representation of the past and planned scan workflow steps at any given time. Planned scan workflow steps maybe subject to change through the scan workflow guidance system and user interaction. Thus, the scan queue could be a special module of the system or it may just be a space in memory where the results could be stored. In an abstract manner, the scan queue represents an order of protocols and the workflow (e.g., additional reconstructions, data analysis) steps. It is typically a central component of a scanner and is usually (but does not have to be) visible in the user interface. The user is preferably able to add protocols to the queue that will be used for scans.

It should be noted that a scan queue is a "hardware" (or at least a memory space) hosting steps of a scan workflow. For this invention only those steps of the scan workflow are important that are hosted in the scan queue. Thus, the expression "scan workflow" should be understood as "at least the steps of the scan workflow hosted or meant to be hosted in the scan queue". When speaking about "modifying a scan work-flow" in this description, it is meant that the steps meant to be included in the scan queue are modified or the scan queue (comprising the scan workflow) is modified. The expression "scan program" could be understood as a program that defines possible scan workflows for an examination.

The scan workflow guidance system lies at the heart of this invention. It is the essential element that allows for patient-specific scan workflow adaptations, while the preconfigured scan protocols and scan programs themselves remain largely standardized by the healthcare provider.

The scan workflow guidance system is designed to consider all or any subsets of the following four input types:
a) The clinical indication(s) for the MRI exam, suspected findings or pathologies (e.g., as hypothesized by the referring physician or indicated by the radiologists) to be fed into the scan UI automatically (e.g., from the RIS) or manually by the MR scanner user.
b) MR images as they are acquired and reconstructed by the MR scanner during the initial scan steps of the MRI exam. The protocols of the initial scan steps may follow a preset standard or be configurable (within certain boundaries) by the MR scanner user. E.g., for a standard neurological MRI examination, the initial scan protocols could be a T2 FLAIR axial measurement and a DWI axial measurement with a suitable parameter set.
c) Metrics in a database of morphological data (e.g., derived from clinical cohort studies) allowing to differentiate between normal and abnormal patient morphologies. The scan workflow guidance system may either possess access to this database or exhibit a built-in, local copy of this database.
d) Sensor data acquired during the scan examination (e.g. ECG, breathing, motion detectors)

A method according to the invention for standardized MRI examinations with patient-centric scan workflow adaptations performed with a system according to the invention comprises the following steps:
- provide a scan protocol to a scan workflow in the scan queue,
- performing a number of scans according to the scan work-flow,
- performing patient-specific adaptions of the scan workflow by the scan workflow guidance system with preconfigured scan protocols and scan programs, based on considerations pertaining to:
   a) clinical indication(s) for the MRI exam, suspected findings or pathologies to be fed into the scan-user-interface automatically or manually by an MR scanner user,
   b) MR images as they are acquired and reconstructed by the MR scanner during the initial scan steps of the MRI exam,
   c) metrics in a database of morphological data allowing to differentiate between normal and abnormal patient morphologies,
   d) sensor data acquired during the scan examination,
- performing a number of scans according to the adapted scan workflow.

In the following, the practical implementation of this method is described.
1) At configuration time (before the examination), suitable (e.g., indication-specific) scan programs are configured (comprising suitably preconfigured scan protocols and/or automated planning features and/or scan workflow guidance features like, e.g., finding detectors). The configuration of scan programs may be performed by the scanner fabricator and/or a master user, e.g., at the site of the healthcare provider. The scan programs could also be selected by a scanner user, however, this scanner user should have enough knowledge about this procedure.
   Instead or in addition to suitable scan programs, a number of suitable scan protocols can be selected.
   This selection is saved in the protocol management system.
   It should be noted that this step is not necessary for the method as long as there are suitable scan programs or protocols already present in the protocol management system.
2) At examination time, the automated or user-induced selection of a preconfigured scan program (or a number of scan protocols) based on, e.g., the patient's scan indication, the scanner performing a number of scan steps (at least one) generating a number of images or physiological data. These scans are performed according to said selection of the initial protocols of the scan program.
3) The acquired image(s) or physiological data being send to the scan workflow guidance system for analysis and preparation of scan workflow recommendation(s).
4) The scan workflow guidance system with preconfigured finding detectors, scan protocols, and scan programs, especially based on considerations pertaining to:
   a) MR images as they are acquired and reconstructed by the MR scanner during the initial scan steps of the MRI exam,
   b) metrics in a database of morphological data allowing to differentiate between normal and abnormal patient morphologies,
   c) sensor data acquired during the scan examination
   d) clinical indication(s) for the MRI exam, suspected findings or pathologies to be fed into the scan UI automatically or manually by an MR scanner user.
5) Optionally, the scan workflow guidance system presenting the recommended scan workflow adaptations to the scanner user for confirmation or modification.
6) the scanner performing a number of n≥0 scans according to the adapted scan workflow.
7) depending on the initially selected scan program and effective scan workflow adaptations, additional variants of steps 3)-6) could be executed by the scanner.

The preparation of the MR examination may consist in an automated, data-based selection of the indication-specific scan program or a user-invoked selection of the patient's scan indication in the UI triggering the loading of the appropriate indication-specific scan program or simply by the user adding a scan protocol (i.e., the "protocol") to an empty scan queue (an empty scan workflow in a scan queue, or in other words: as scan workflow steps). However, the preparation of the MR examination may also comprise actions like "selecting a number of protocols from the database of the protocol management system and arranging them in the scanner queue", "modifying selected protocols", "adding scan programs to the scanner queue" (a scan program is a routine preferably comprising suitably preconfigured scan protocols and/or automated planning features and/or scan workflow guidance features), wherein these steps could also be performed by a master user at configuration of the system. It is only required that there actually is a scan protocol in the initial scan queue and the scan workflow guidance system is configured. Ideally, the configuration of the scan program selected at the start of the MR examination is performed at a configuration time.

Then, based on the modified scan workflow, a number of scans are performed (the scans accord to the protocols of the scan workflow).

Then, there are some results generated by the scan workflow recommendation system from the data acquired by the scanner. These results may be based on an analysis of the MR images as they are acquired and reconstructed by the MR scanner during the initial scan steps of the MRI exam (the scan), metrics in a database of morphological data allowing to differentiate between normal and abnormal patient morphologies, or sensor data acquired during the scan examination. For example, images taken during a scan could be examined (automatically or by a user) and the outcome of the examination (e.g., a finding) is the result.

Based on these results, patient-specific adaptions of the scan workflow may be generated by the scan workflow guidance system with preconfigured scan protocols and scan programs. This means, that protocols of the scan workflow yet not applied to the MRI system are adapted based on the results. Alternatively or additionally, new predefined protocols could be automatically fetched from the database of the protocol management system and added to the scan workflow.

Then, the scan workflow (in the scan queue) may be continued by performing a number of scans according to the adapted scan workflow. However, the exam can also be stopped at this point, especially when there are no findings detected.

It should be noted that also, the repetition of a previous protocol could be applied, since it could happen, that a post contrast agent scan was run, the system found out that the injector didn't work or contrast agent hasn't arrived in the tissue yet (no contrast agent visible in the image) and therefore the same protocol is run again or added to the workflow right behind the protocol just been applied.

The added scan protocols may be tagged with priority labels according to the prior assessment of the healthcare provider as setup in the scan protocol management system.

It is preferred that once a user has initially started a scan workflow execution, e.g., by selecting a scan indication, the scan queue is created automatically with minimal user interaction.

At each time point during the MR examination, the scan queue holds a representation of all past and planned scan steps. While past scan steps remain in the queue as historical record, planned steps in the queue may be subject to change at any point in time before execution. The queue occupies, e.g., a memory unit, or a software-unit, e.g., a virtual memory space, e.g., on the scanner, on an edge device or in the cloud. The scan workflow represents the abstract sequence of actions (i.e., scan protocols, scan programs, and possibly other suitable actions) that could be performed during an examination. Thus, the workflow describes the order of protocols (e.g., first T1, then T2, then DWI), while the scan queue also stores the necessary protocol parameters for each specific protocol, so that the scanner can execute it as soon as it hits this step. However, for the invention, the work-flow could nevertheless be regarded as the workflow steps being present in the scan queue.

However, it is preferred that a user may always (but does not have to) manually interact and remove and/or add and/or modify the protocols (of the scan workflow). Therefore, an access to the central database should be available, but the main focus is on automation.

In practice, it is important, to strictly distinguish between the scanner user (operator of the scanner at exam time) and the master-user (experienced technologist or radiologist, who defines and preconfigures the protocols, programs, and scan workflow adaptation options at configuration time). In a special case, a scanner user could also be a master-user, but in general they are two different roles and use different UIs for their tasks. A scanner user should have less access to modifications of the programs, protocols or the scan queue than a master user.

In summary, the concept presented in this invention allows the healthcare provider to setup a consistent and comprehensive set of indication-driven, preconfigured MR scan workflows covering their entire scanner fleet. If the scan indication and patient specifics are provided to the MR scanner electronically, the standardized preconfigured MR scan workflows can be employed by the MR scanner requiring no manual intervention at the scanner. Alternatively, the MR scanner user can select the appropriate standardized scan program by inserting the scan indication and optional patient specifics.

Preferably all patient-specific scan workflow modifications are identified by the MR scanner internally and can be directly used for the automated completion of the entire scan workflow. If preferred by the healthcare provider, additional reviewing and modification steps through the MR scanner user are possible, but not required.

A scan workflow guidance system according to the invention is adapted for a system according to the invention and, thus, configured to use a preconfigured scan protocol and/or scan program with the capability to perform patient specific adaptions, wherein the scan protocols and/or scan programs, which are selected by a master user, are getting adapted based on acquired data, e.g. image data, and/or (master) user selected presetting.

A control device according to the invention for controlling a magnetic resonance imaging system comprises a system according to the invention. Especially, it is designed to perform the method according to the invention. The control device may comprise additional units or devices for controlling components of a magnetic resonance imaging system, e.g., a sequence control unit for measurement sequence control, a memory, a radio-frequency transmission device that generates, amplifies, and transmits RF pulses, a gradient system interface, a radio-frequency reception device to acquire magnetic resonance signals, and/or a reconstruction unit to reconstruct magnetic resonance image data.

A magnetic resonance imaging system comprises a system according to the invention, especially a control device according to the invention.

Some units or modules of the system or the control device mentioned above can be completely or partially realized as software modules running on a processor of a system or a control device. A realization largely in the form of software modules can have the advantage that applications already installed on an existing system can be updated, with relatively little effort, to install and run these units of the present application. The object of the invention is also achieved by a computer program product with a computer program that is directly loadable into the memory of a device of a system or a control device of a magnetic resonance imaging system, and which comprises program units to perform the steps of the inventive method when the program is executed by the control device or the system. In addition to the computer program, such a computer program product can also comprise further parts such as documentation and/or additional components, also hardware components such as a hardware key (dongle etc.) to facilitate access to the software.

A computer readable medium such as a memory stick, a hard-disk or other transportable or permanently-installed carrier can serve to transport and/or to store the executable parts of the computer program product so that these can be read from a processor unit of a control device or a system. A processor unit can comprise one or more microprocessors or their equivalents.

Particularly advantageous embodiments and features of the invention are given by the dependent claims, as revealed in the following description. Features of different claim categories may be combined as appropriate to give further embodiments not described herein.

As said above, the fundamental idea of the invention is to start with an indication-specific set of initial scan protocols (a number of n protocols with n > 0). When the data acquired by applying these initial protocols to an MRI system are analyzed, findings are detected. The analyzation can be achieved by "finding detectors", i.e., units specially designed for detecting findings in the images, wherein some suitable finding detectors are already known in the art.

Based on the findings, the scan workflow may be adapted and/or extended by the scan workflow guidance system. This way, the workflow that initially starts in a standardized way ultimately becomes disease as well as patient specific.

Components (like finding detector algorithms or customer-specific configurations) are preferably hosted centrally in the system, the healthcare provider's IT infrastructure or a cloud service to make standardization across a fleet of scanners easier, but they could be deployed locally.

Thus, the invention allows a healthcare provider to generate individual, indication-specific, standardized scan workflows that (based on the machine acquired data and automatically extracted patients' specific findings and requirements) adapt a scan queue at examination time, in a way that can be preconfigured according to the guidelines of the healthcare provider. In short: The invention automatically generates a patient-specific workflow out of pre-defined protocols during examination time.

The sequence of protocols is preferably generated by a scan queue manager, preferably hosted by the scan workflow guidance system. The management of the healthcare provider's preferred protocols to be inserted by the scan workflow guidance system is preferably managed by the protocol management system, where disease specific programs are stored.

The initial protocols could be inserted manually into the queue (healthcare provider often like to have all options), but they don't have to be. They can be preconfigured just like the scan workflow adaptation logic can be preconfigured by the healthcare provider.

Based on the results of the scans (with the protocols in the scan workflow) one by one, additional protocols are automatically retrieved, or protocols in the scan queue are removed or modified.

As an example: a patient is referred to MR with suspicion of stroke. The system automatically pulls a stroke program from the central location, which contains a diffusion and a hemorrhage protocol. After the diffusion protocol it may be determined that there is no stroke and the hemorrhage protocol is removed and instead regular "brain standard" protocols are pulled in to possibly identify other sources of the clinical findings that lead to the suspicion of stroke. In case a diffusion abnormality is found, the hemorrhage scan is run to see, what type of stroke this is and additional protocols to determine the age of the stroke are pulled in. If in the hemorrhage scan it is observed that the patient is moving and image quality is degraded, imaging parameters of the sequence are adapted (e.g., motion correction turned on) and the subsequent protocols are also modified to account for this observation.

According to a preferred system, the protocol management system comprises a centralized database for storing protocols. It is preferred that the protocol management system comprises a default set of scan protocols and/or scan programs stored in a centralized, operation-wide accessible database. The access to the database is preferably depending on certain access rights of user groups. Thus, the protocol management system preferably offers a tiered access control.

It is preferred that the set of scan protocols and/or scan programs is linked to a protocol identifier, especially with the information selected from the group
- sequence with preconfigured parameter sets,
- reconstruction with preconfigured parameter sets,
- automated quality assurance steps,
- post-processing operations to be performed on acquired images and
- other relevant parameters.

Said protocol identifier is ideally a globally unique identifier (GUID). Alternatively, it could be a local identifier, e.g. a number, an address or string name, preferably given by the healthcare provider.

Ideally, the protocol management system should be associated with a suitable scan protocol editor that allows the healthcare provider's central protocol configurators to conveniently edit, configure and manage a large set of scan protocols.

The latter could be setup to allow the protocol configurator (usually a master user) to compose scan programs from preconfigured modules. The configurator may assemble the scan programs choosing from a set of, e.g., suitably preconfigured scan protocols, automated planning features and scan workflow guidance features. One preferred variant of the scan protocol editor would allow the customer to compose their scan protocols from a toolbox of fundamental building blocks (e.g., sequences, reconstructions, and appropriate parameter sets). The healthcare provider may use the scan protocol & program editor to preconfigure their own standardized smart modules comprised of preconfigured scan protocols, automated planning, and quality assurance steps.

In the scan program editor, the set of smart modules as well as more fundamental elements inside the toolboxes are preferably employed to assemble the scan program in a subsequent step. In the scan program editor, the healthcare provider can preferably define up to n initial smart modules (e.g., preconfigured protocols with auxiliary smart components (e.g., automated slice planning, quality assurance steps) for each scan step), especially from respective scan workflow toolboxes. This allows a healthcare provider to setup their scan protocol standards and derived scan programs in a modular fashion for each clinical indication.

A smart module typically comprises a scan protocol and additional components, preferably an auto planner and/or an automatic quality assessment module. In short, it could be said that a smart module comprises a scan protocol and an additional functionality concerning image acquisition planning or quality assurance of this scan protocol. The latter may be performed with the help of intelligent algorithms (e.g., machine learning, deep-learning or classical, rule-based algorithms). Thus, smart modules can be preconfigured scan protocols, automated planning and quality assurance steps. The expression "Smart" refers to their property containing analytical tools from a "Smart Component Toolbox". These may be powered by, e.g., rule-based algorithms, machine learning or deep learning algorithms, generally perceived as smart or intelligent.

Thus, according to a preferred system, the protocol management system comprises a scan protocol editor and/or a scan program editor, each with different building blocks.

Preferably, a scan protocol can be contained in different modules, preferably wherein the scan protocol editor is designed to edit, configure and manage the set of scan protocols comprised by the protocol management system, especially designed for composing scan protocols from a toolbox of fundamental building blocks. Alternatively or additionally, the scan program editor is designed to assemble a scan program with a set of smart modules comprised by elements of different toolboxes.

Optionally, a set of finding detectors can be added after the n-th initial scan protocol has finished. The finding detectors will check the acquired data for the appearance of specific features (i.e., findings) and add, replace or remove preconfigured scan steps to/in/from the scan queue based on the type of identified findings.

Thus, according to a preferred method, a set of finding detectors is added after the n-th initial scan protocol has finished, the finding detectors checking the acquired image for the appearance of specific features and adding, replacing or removing preconfigured scan steps to/in/from a scan queue based on the type of identified findings, preferably wherein a scan protocol editor and/or a scan program editor allow a healthcare provider to define which protocols or smart modules should be added, replaced or removed to/in/from a scan queue manager in case the finding detectors make a specific observation.

The scan protocol and program editor allows the healthcare provider to define which protocols or smart modules should be added, replaced or removed to/in/from the scan queue manager in case the finding detectors make a specific observation.

Additional meta-information (e.g., priority of the added scan module) may preferably be configurable for each element for consideration by the scan queue manager. The finding detectors may also take additional input into account (e.g., input from temperature, electrocardiogram (ECG), pulse, or motion, blood pressure or other vital sign sensors), or other patient information (e.g., lab data, prior images, clinical data) to improve decisions on the workflow.

Preferably, the protocol management system is designed such that at least a master user is able to define what protocols and what workflow combinations and adaptations are possible. It is preferably further defined such that during the execution of the scan workflow, the system is able to just pulling the pre-defined options and reacting on the patient conditions.

At purchase of a scanner, scan protocols and scan programs are usually provided fully configured and ready to use as part of the MR scanner software. If the healthcare provider does not need any modifications, the scan protocols and scan programs could be used 'as is' for the protocol management system. Usually, healthcare providers want to modify scan protocols and or programs to comply with their preferences and or official guidelines.

The scan protocol editor described above is designed for configuring protocols. Certainly, the protocols can also be set up on the scanner or in a stand-alone environment and then "manually" copied to the central location. The possible combinations and adaptations could also be configured/defined on a script or file-basis.

The scan program editor described above is preferably designed for configuring the possible workflow combinations and decision points. The possible combinations and adaptations could also be configured on a script or file-basis on the central location. Especially for long-term maintenance and a larger scanner fleet local and/or manual management of scan programs may be unpractical. Therefore, a centralized scan protocol and program editing and management system would be of big practical utility.

According to a preferred system, the scan workflow guidance system is configured to consider all or any subsets of the following four input types:
a) The clinical indication(s) for the MRI exam, suspected findings or pathologies (e.g., as hypothesized by the referring physician or indicated by the radiologists) to be fed into the scan UI automatically (e.g., from the RIS) or manually by the MR scanner user.
b) MR images as they are acquired and reconstructed by the MR scanner during the initial scan steps of the MRI exam. The protocols of the initial scan steps may follow a preset standard or be configurable (within certain boundaries) by the MR scanner user. E.g., for a standard neurological MRI examination, the initial scan protocols could be a T2 FLAIR axial measurement and a DWI axial measurement with a suitable parameter set.
c) Metrics in a database of morphological data (e.g., derived from clinical cohort studies) allowing to differentiate between normal and abnormal patient morphologies. The scan workflow guidance system may either possess access to this database or exhibit a built-in, local copy of this database.
d) Sensor data acquired during the scan examination (e.g., ECG, breathing, motion detectors).

Preferably, the scan workflow guidance system exhibits a modular architecture of mutually independent analysis branches. Each analysis branch preferably comprises an image analysis module (especially each comprised of, e.g., one or a combination of several deep convolutional neural nets or machine learning based pattern detection algorithms) able to detect one or a specific combination of relevant features from the input MR image and can combine this with other features.

Regarding finding detectors and analysis modules, these expressions mean slightly different embodiments. An image analysis module is a software component that analyzes images for various purposes. A finding detector analyses data (e.g., images or physiological data, like ECG or blood pressure) to detect specific features, signatures or finding on it. There is a non-vanishing overlap between the two classes.

Preferably, the scan workflow guidance system comprises a set of relevant feature detectors associated with the set of image analysis modules that is especially suitably defined as a set of different detectors for different classes of radiological findings, pathologies, image artifacts, or diseases.

The set of relevant feature detectors (associated with the set of image analysis modules) may be suitably defined as a set of different detectors for different classes of, e.g. radiological findings, pathologies, image artifacts, or diseases.

In the following we provide example sets of detector classes for the application of neurological MR scan workflows:
Example 1: radiological finding classes: hemorrhage, ischemia, lesion, white matter hyperintensities, degenerative structures, unknown anomalies or pathologies, morphological anomalies.
Example 2: classes of frequent, incidental findings: cysts, structural vascular abnormality, other developmental variations, infarctions, white matter hyper-intensities, micro-hemorrhages, parenchymal calcification, tumors, other pathologies.
Example 3: classes of frequent pathologies: hemorrhage, lesion, multiple sclerosis (MS), dementia, unspecific anomalies.
Example 4: artifact classes: motion artifacts, implant artifacts, aliasing artifact etc.

In case the image analysis module in each analysis branch detects a positive case, it may (either directly or following further dissemination/characterization of the detected feature) trigger the output, replacement or deletion of one or a set of preconfigured scan protocols (incl. appropriate parameters) to/in/from the scan queue manager. Ideally, each feature detector is equipped with confidence metric to determine how certain the feature has been detected). In order to properly determine the confidence of the prediction auxiliary analysis modules might be helpful or required (e.g., image quality detection modules).

Alternatively, all or only certain user groups (exhibiting advanced user rights) may be allowed to manually trigger any or all of the finding detectors by selecting the respective findings in the scan UI, such that the corresponding scan protocols will be forwarded to a scan queue manager.

The added scan protocols are preferably tagged with priority labels according to the prior assessment of the healthcare provider as setup in the scan protocol management system.

A preferred scan workflow guidance system comprises a scan queue manager designed to curate a set of scan workflow adaptation as output from the analysis branches. This scan queue manager is a very advantageous and sometimes important module of the scan workflow guidance system. It has the central task to curate the set of scan workflow adaptation as output from the analysis branches.

The scan queue manager is preferably designed to achieve one or more tasks. Among these tasks we see:
- to curate the set of added protocols (e.g., removing protocol duplicates, merging similar protocols),
- performing consistency checks to ascertain overall scan workflow consistency (e.g., ascertaining that pre and post contrast measurement steps are properly sorted in time with respect to contrast injection or newly introduced parameter settings are consistent across different protocol steps),
- ensuring given time constraints on the available scan work-flow slot (e.g., by considering priority levels of added protocol steps and ascertaining proper coverage of scan workflow elements as predefined in the scan protocol management system) .

Thus, the scan queue manager consolidates the results that come back from the analysis modules and modifies the queue accordingly. The potential protocols are stored in the protocol management system.

After each scan session a report is preferably created by the scan workflow guidance system. These reports provide information regarding the initially selected workflow steps, provided suggestions incl. the basis for these suggestions and executed protocol steps incl. parameter settings as well as the executed protocol steps incl. parameter settings, entered comments, and scan times. These reports could also include DICOM objects which might need to be appropriately anonymized/de-identified according to the locally applicable data privacy law.

In an additional, preferred variant, the scan workflow guidance system reports the suggested and/or executed scan work-flow to be archived and accessible in the protocol manager. These reports are preferably accessed via an analysis tool providing insights into protocol usage, circumstances under which the suggestions of the scan workflow guidance system were followed vs. not followed, and a large set of statistical data derived from the scanner's scan history across the institutions scanner fleet.

It is preferred that a user is able to confirm or change the scan workflow recommendations of the scan workflow guidance system before execution.

According to a preferred system, the user interface is configured to review and/or edit the scan workflow, especially to insert the scan indication or suspected pathologies of the patient, preferably in a tiered privilege level concept.

Preferably, the user interface allows the user to add a set of standard protocols of the respective application, regardless of the recommendations determined by the scan workflow guidance system downstream, especially wherein the user interface is equipped with an option to review and edit scan workflow adaptation as suggested by the scan workflow guidance system.

It should again be noted that "scan workflow" here means "scan workflow steps in the scan queue".

Preferably, the scan UI is designed to be or to comprise a master user interface, which allows a master user to perform preconfigurations to scan protocols, scan programs and/or automated scan workflow modifications.

Preferably, the scan UI is designed to be or to comprise a user-scanner interface developed to display, e.g., all or some of the following exam concerning information: the planned, preconfigured and adapted scan queue, acquired images, scan workflow recommendations from the scan workflow guidance system.

Regarding the scan queue, it is preferably only one queue that, prospectively seen, displays the planned scan steps and, retrospectively seen, displays the history of all actually executed scan steps. It preferably comprises acquisition protocols and workflow steps. Based on the disease suspicion, an initial set of protocols (can be even only one protocol) and workflow steps can be added to the scan queue. Having the additional information using the workflow step(s), the system can then modify the remaining scan queue. This can happen again after each protocol that is scanned.

Preferably, all protocols are set up by a master user (e.g., the lead technologists) centrally, the selection for the individual patient is done based on the suspected disease (indication) and especially prescribed by the radiologist. The scan UI may help the scanner user to retrieve the right program.

The reason to have initial protocols is at first hand, to acquire images or data that are required in any case and secondly to generate a first data input for the scan workflow guidance system. The minimum number of initial scan protocols could be one, if the scan workflow guidance system only considers image data as input. If the scan workflow guidance system additionally considers data form scanner-connected physio sensors (e.g., ECG, respiratory sensor), such signals could already trigger scan workflow adaptions without any initial protocol.

Modifications to the queue and the parameters of the protocols can only be done for those protocols, which have not been scanned yet. However, the decisions for modification can happen after each protocol, so there are not only two parts, but in principle there could be 1 to m-parts of the scan queue.

In a preferred system according to the invention, components of the system are part of a data-network, wherein preferably the data-network and a medical imaging system (i.e., the magnetic resonance imaging system which provides image data) are in data-communication with each other, wherein the data-network preferably comprises parts of the internet and/or a cloud-based computing system, wherein preferably the system according to the invention or a number of components of this system is realized in this cloud-based computing system. For example, the components of the system are part of a data-network, wherein preferably the data-network and a medical imaging system which provides the image data are in communication with each other. Such a networked solution could be implemented via an internet platform and/or in a cloud-based computing system.

The method may also include elements of "cloud computing". In the technical field of "cloud computing", an IT infrastructure is provided over a data-network, e.g., a storage space or processing power and/or application software. The communication between the user and the "cloud" is achieved by means of data interfaces and/or data transmission protocols.

In the context of "cloud computing", in a preferred embodiment of the method according to the invention, provision of data via a data channel (for example a data-network) to a "cloud" takes place. This "cloud" includes a (remote) computing system, e.g., a computer cluster that typically does not include the user's local machine. This cloud can be made available in particular by the medical facility, which also provides the medical imaging systems. In particular, the image acquisition data is sent to a (remote) computer system (the "cloud") via a RIS (Radiology Information System) or a PACS (Picture Archiving and Communication System).

Within the scope of a preferred embodiment of the system according to the invention, the abovementioned units (especially the protocol management system and/or the scan workflow guidance system) are present on the "cloud" side. A preferred system further comprises, a local computing unit connected to the system via a data channel (e.g., a data-network, particularly configured as RIS or PACS). The local computing unit includes at least one data receiving interface to receive data. Moreover, it is preferred if the local computer additionally has a transmission interface in order to send data to the system.

The combination of features results in the following benefits:
Scan protocols can be setup, maintained, optimized and analyzed centrally by the healthcare provider to establish and maintain an operation-wide protocol standard for each scan indication. This way, unwarranted variations in the choice of scan protocols by the MR scanner can be prevented systematically.

The preconfigured scan protocols and build-in automation of previously manual interactions of the MR scanner user with the scan software prevents unwarranted variations in scan protocol parameters.

The build-in automated slice planning and quality assurance for each scan step renders previously manual planning and control efforts of the MR scanner user unnecessary, and thus warranting standardized image outcomes.

Patient-centric scan workflow adaptations are possible through build-in image analysis and scan workflow suggestion of additional scan steps according to the healthcare provider's standards.

Other objects and features of the present invention will become apparent from the following detailed descriptions considered in conjunction with the accompanying drawings. It is to be understood, however, that the drawings are designed solely for the purposes of illustration and not as a definition of the limits of the invention.
Figure 1 shows a simplified MRI system comprising a system according to an embodiment of the invention.
Figure 2 shows a block diagram of the process flow of a preferred method according to the invention.
Figure 3 shows a possible realization of a preferred simple protocol database.
Figure 4 shows a preferred protocol management system.
Figure 5 shows an example of a system according to an embodiment of the invention.

In the diagrams, like numbers refer to like objects throughout. Objects in the diagrams are not necessarily drawn to scale.

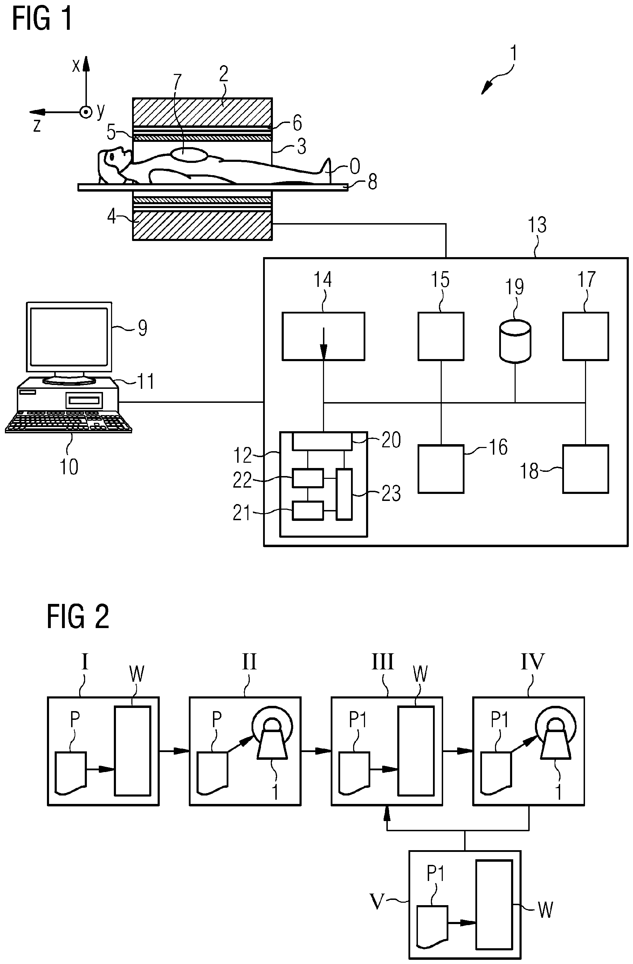

Figure 1 shows a schematic representation of a magnetic resonance imaging system 1 ("MRI-system"). The MRI system 1 includes the actual magnetic resonance scanner (data acquisition unit) 2 with an examination space 3 or patient tunnel in which a patient or test person is positioned on a driven bed 8, in whose body the actual examination object is located.

The magnetic resonance scanner 2 is typically equipped with a basic field magnet system 4, a gradient system 6 as well as an RF transmission antenna system 5 and an RF reception antenna system 7. In the shown exemplary embodiment, the RF transmission antenna system 5 is a whole-body coil permanently installed in the magnetic resonance scanner 2, in contrast to which the RF reception antenna system 7 is formed as local coils (symbolized here by only a single local coil) to be arranged on the patient or test subject. In principle, however, the whole-body coil can also be used as an RF reception antenna system, and the local coils can respectively be switched into different operating modes.

The basic field magnet system 4 is designed that images can be recorded. It here is designed in a typical manner so that it generates a basic magnetic field in the longitudinal direction of the patient, i.e., along the longitudinal axis of the magnetic resonance scanner 2 that proceeds in the z-direction. The gradient system 6 typically includes individually controllable gradient coils in order to be able to switch (activate) gradients in the x-direction, y-direction or z-direction independently of one another.

The MRI system 1 shown here is a whole-body system with a patient tunnel into which a patient can be completely introduced. However, in principle the invention can also be used at other MRI systems, for example with a laterally open, C-shaped housing, as well as in smaller magnetic resonance scanners in which only one body part can be positioned.

Furthermore, the MRI system 1 has a central control device 13 that is used to control the MRI system 1. This central control device 13 includes a sequence control unit 14 for measurement sequence control. With this sequence control unit 14, the series of radio-frequency pulses (RF pulses) and gradient pulses can be controlled depending on a selected pulse sequence based on a certain protocol P (i.e., a scan protocol P) or, respectively, a series of multiple pulse sequence based on a certain protocol P to acquire magnetic resonance images within a measurement session. Different control protocols P for different measurements or measurement sessions are typically stored in a memory 19 and can be selected by and operator (and possibly modified as necessary) and then be used to implement the measurement.

To output the individual RF pulses of a pulse sequence PS, the central control device 13 has a radio-frequency transmission device 15 that generates and amplifies the RF pulses and feeds them into the RF transmission antenna system 5 via a suitable interface (not shown in detail). To control the gradient coils of the gradient system 6, the control device 13 has a gradient system interface 16. The sequence control unit 14 communicates in a suitable manner with the radio-frequency transmission device 15 and the gradient system interface 16 to emit the pulse sequence.

Moreover, the control device 13 has a radio-frequency reception device 17 (likewise communicating with the sequence control unit 14 in a suitable manner) in order to acquire magnetic resonance signals (i.e., raw data) for the individual measurements, which magnetic resonance signals are received in a coordinated manner from the RF reception antenna system 7 within the scope of the pulse sequence.

A reconstruction unit 18 receives the acquired raw data and reconstructs magnetic resonance image data therefrom for the measurements. This reconstruction is typically performed on the basis of parameters that may be specified in the respective measurement or control protocol. For example, the image data can then be stored in a memory 19.

Operation of the central control device 13 can take place via a terminal 11 with an input unit 10 and a display unit 9, via which the entire MRI system 1 can thus also be operated by an operator. MR images can also be displayed at the display unit 9, and measurements can be planned and started by means of the input unit 10 (possibly in combination with the display unit 9), and in particular suitable control protocols can be selected (and possibly modified) with suitable series of pulse sequence as explained above.

The control device 13 comprises a system 12 designed to perform the method according to the invention. This system 12 comprises the following components that may appear to be software modules. In this simple example, the system 12 comprises the following components.

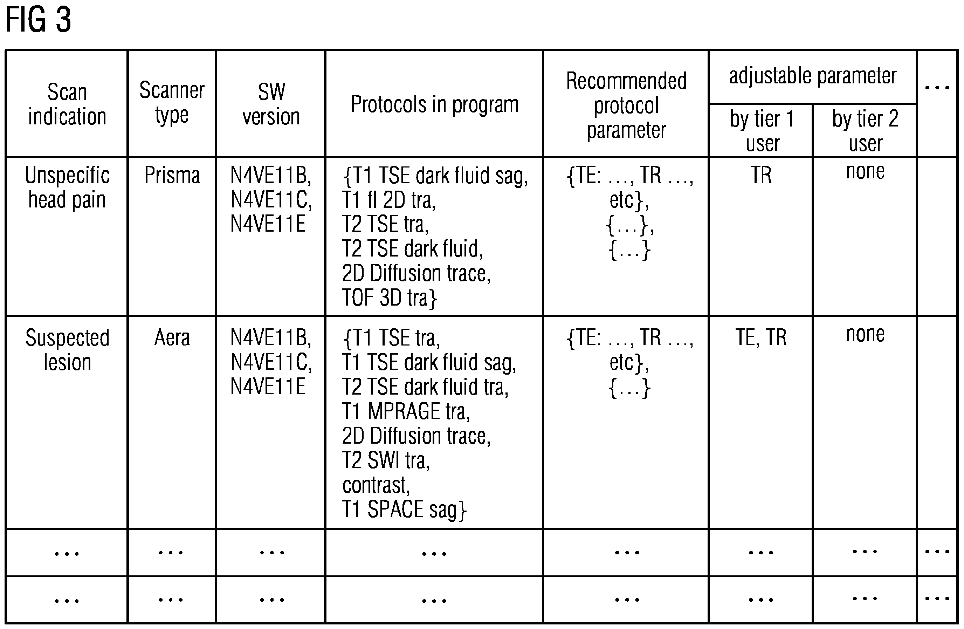

A scan-user-interface 20, that is configured to modify a scan workflow W (see e.g., figure 3) by a user. In this example, the scan-user-interface 20 is a simple data-interface that is able to provide a graphical user interface (GUI) for the terminal 11. The GUI could be shown on the display unit 9 and a user may provide input data for the scan-user-interface 20 via the input unit 10. In this example, the scan-user-interface 20 is configured to review and/or edit the scan workflow W, especially to insert the scan indication or suspected pathologies of the patient, preferably in a tiered privilege level concept. It is preferred that the scan-user-interface 20 allows the user to add a set of standard scan protocols P of the respective application, regardless of the recommendations determined by the scan workflow guidance system 22 downstream, especially wherein the scan-user-interface 20 is equipped with an option to review and edit scan work-flow W adaptation as suggested by the scan workflow guidance system 22.

A protocol management system 21 is designed for setup and/or maintain scan programs Pr and/or scan protocols P centrally. It here comprises a plurality of scan protocols P that may be used for different scanning purposes.

A scan workflow guidance system 22 is the heart of the system 12 and is configured to use a preconfigured scan protocol P and/or scan program Pr by patient specific adaptions (i.e. to suggest or execute patient specific adaptions). The scan protocols P and/or scan programs Pr, which are selected by a user, are getting adapted based on image data and/or user selected presetting. Here, the most important steps of the method of figure 2 are performed.

All of the above components, scan-user-interface 20, protocol management system 21 and scan workflow guidance system 22 are in this example able to add data (e.g., protocols or adjustment data) to the scan queue 23, where the scan workflow W is hosted. The scan queue 23 in this example is designed to provide a representation of the past and planned scan workflow steps at any given time, preferably wherein planned scan workflow steps may be subject to change through the scan workflow guidance system 22 and user interaction.

The MRI system 1 according to the invention, and in particular the control device 13, can have a number of additional components that are not shown in detail but are typically present at such systems, for example a network interface in order to connect the entire system with a network and be able to exchange raw data and/or image data or, respectively, parameter maps, but also additional data (for example patient-relevant data or control protocols).

The manner by which suitable raw data are acquired by radiation of RF pulses and the generation of gradient fields, and MR images are reconstructed from the raw data, is known to those skilled in the art and thus need not be explained in detail herein.

Figure 2 shows a block diagram of the process flow of a preferred method according to the invention for standardized MRI examinations with patient-centric scan workflow W adaptations performed with a system 12 according to the invention (see. the other figures).

In step I, a scan workflow W is modified by a user, by adding a protocol P to it.

In step II, a number of scans according to the scan workflow W are performed by applying a respective number of scan protocols P to an MR system 1. For example, the scan protocol P added by the user in step I is applied to the MR scanner.

In step III, patient-specific adaptions of the scan workflow W are performed by the scan workflow guidance system 22 with preconfigured scan protocols P and scan programs Pr. These adaptions concern amendments of scan protocols P already present in the scan workflow W or including additional scan protocols P to the scan workflow W, wherein the adaptions are based on considerations pertaining to:
a) clinical indication(s) for the MRI exam, suspected findings or pathologies to be fed into the scan-user-interface (20) automatically or manually by an MR scanner user,
b) MR images as they are acquired and reconstructed by the MR scanner during the initial scan steps of the MRI exam,
c) metrics in a database of morphological data allowing to differentiate between normal and abnormal patient morphologies,
d) sensor data acquired during the scan examination.

A set of finding detectors F may check the acquired images for the appearance of specific features and add, replace or remove preconfigured scan steps to/in/from the scan workflow W based on the type of identified findings.

In this example, an additional scan protocol P1 is added to the scan workflow W.

In step IV, a number of (further) scans according to the adapted scan workflow W are performed. Here, the additional scan protocol P1 is applied to the MR system 1. This step may be repeated several times until the scan queue 23 is finished.

In step V, the scan workflow guidance system 22 reports a suggested and/or executed scan workflow W to be archived and accessible in the protocol management system 21. With "new" a scan protocol is meant that is jet not comprised by the protocol management system 21. This step may be performed anytime a new scan protocol P, P1 is present. In this example, prior application it is tested whether the added scan protocol P1 is already present in the protocol management system 21 (it could be manually adapted by a user) and if yes, it is saved in the protocol management system 21.

Figure 3 shows a possible realization of a preferred simple protocol database (could also be regarded as a simple protocol management system 21) in form of a table with attribute columns. In this example, the table hosts information about the scan indication, the scanner type, the software (SW) version, the protocols in program connected with the scan indication, recommended protocol parameters and parameters that may be adjusted by a user. The shown table offers alternatives of adjustable parameters for different types of users ("tier 1 user" and "tier 2 user") having different access rights. The dots indicate that there may be more columns and/or lines.

The column "scan indication" is drawn to certain diseases, e.g., unspecific head pain or suspected lesion as shown in the figure. It should be noted that certain diseases may be present multiple times, e.g., connected with different scanner types (possibly resulting in different protocols to be used).

The column "scanner type", e.g., "Prisma" scanner or "Aera" scanner offers the possibility to distinguish between different scanner types. As already said above, the suggested protocol may depend on the used scanner type, even if examining a similar disease.

The column "SW version", e.g., N4VE11B, N4VE11C, N4VE11E offers information about the software version. Depending on the SW version, possibly different protocols or parameter settings have to be used. In the shown case, this column only offers additional information.

The column "protocols in program" comprises the actual protocols to be used for an examination connected with the scan indication of the certain row. There are shown two protocols for the two exemplary diseases (with the two different scanner types). 'T1' and 'T2' mean the contrasts to be measured, 'TOF' the time of flight with certain orientations ('tra' or 'sag'). There are special sequences /acquisition techniques such as 'dark fluid', 'turbo spin echo' (TSE) '2D Diffusion trace', 'FLASH (fl) 2D', 'MPRAGE', 'SWI' or 'SPACE'.

The next column "recommended protocol parameters" comprises a list of parameters of the protocol of the certain row that may be adjusted or predefined by a user or an automatic routine (also a predefined list). Here, the echo time TE and the repetition time TR are listed, but there may also be other parameters in this list.

The columns "adjustable parameter" comprise a number of parameters, especially selected from the column "recommended protocol parameters", that may be adjusted by a user depending on the access rights of the user. Here, two different user groups with different access rights are defined "tier 1 users" may amend or adjust certain parameters, "tier 2 users" are not allowed to change any parameter.

Although, the management of protocols may be realized with a simple table, a more elaborated protocol management system is preferred.

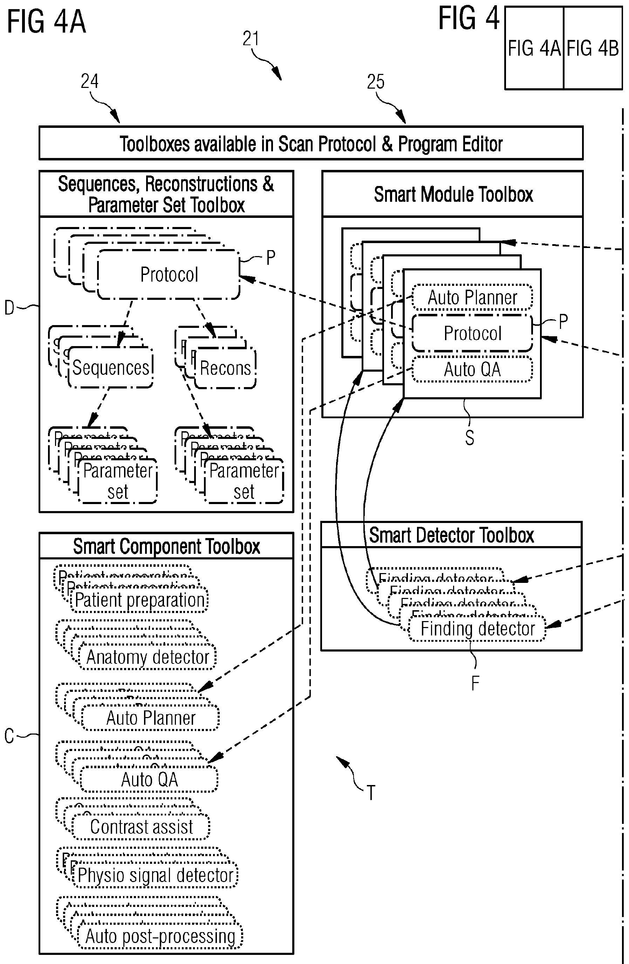

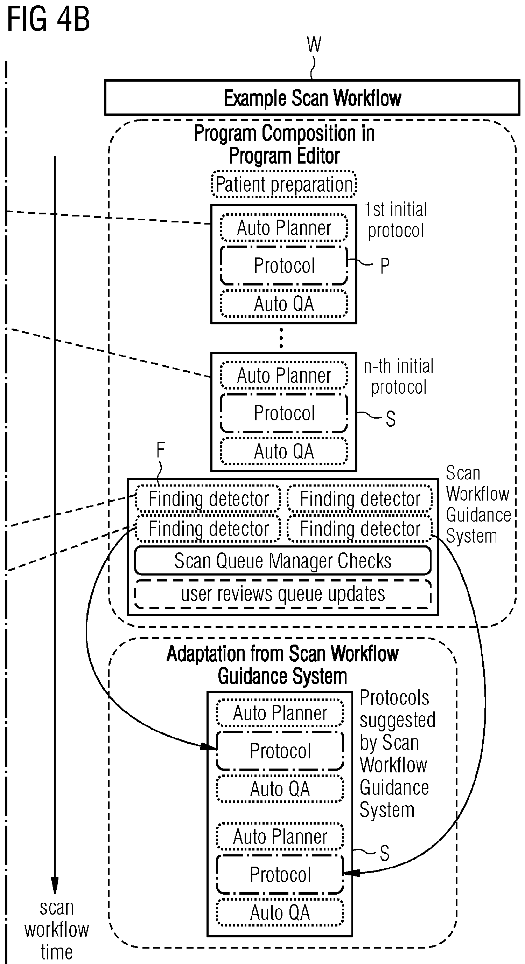

Figure 4 shows a preferred protocol management system 21 in form of a schematic exhibiting the configuration and customization options offered by the protocol management system 21.

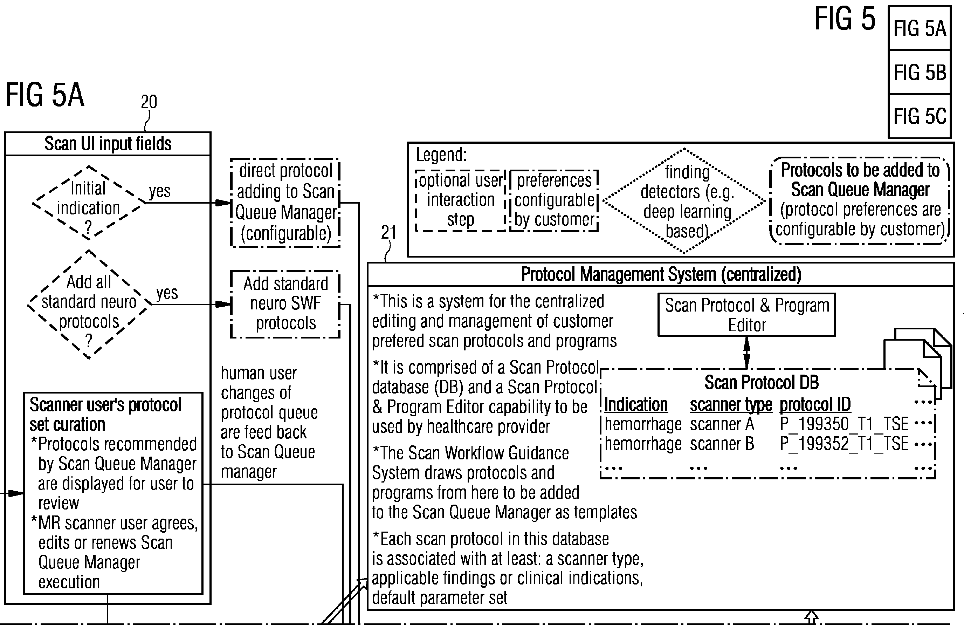

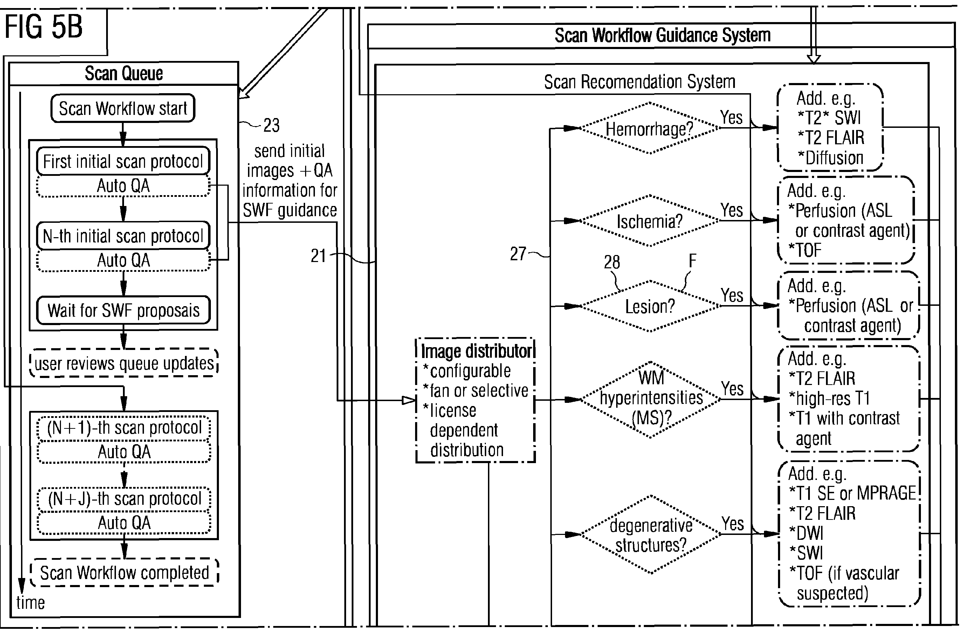

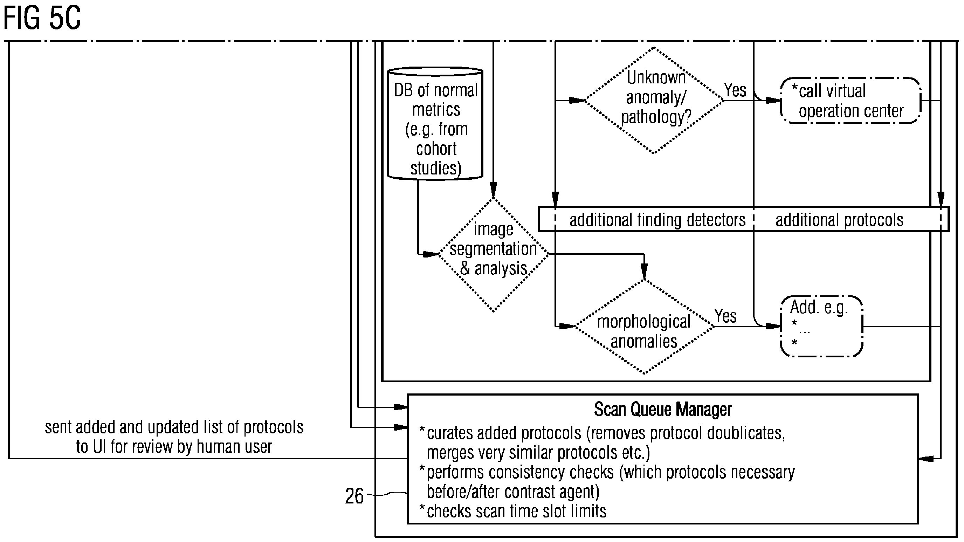

The right side of figure 4 ("Example Scan Workflow") shows a detailed view of the steps that would be performed in the scan queue 23 (see figure 5) for a specific disease. The upper part ("Program Composition in Program Editor") was configured by a user, the lower part (Adaptions from Scan Work-flow Guidance System") includes the planned execution of data analysis and was generated by the scan workflow guidance system 22.

The left side of this figure shows toolboxes T available in the scan protocol editor 24 & scan program editor 25. One toolbox T comprises the protocols P used by the invention. In this example, it is a database D and may in a simple case solely form the protocol management system 21. However, in the shown case, the protocol management system 21 has a more complex structure.

In a smart module S toolbox T a user or the scan workflow guidance system 22 may find already configured models comprising a protocol P and also an auto planner and an automatic quality assurance module ("Auto QA"). The additional modules, smart components C, are fetched from the "Smart Component Toolbox" (lower left). The "Smart Detector Toolbox" comprises finding detectors F that are able to automatically fetch suitable configured models based on findings in images recorded by a protocol P of the scan workflow W. As it can be seen, a user has arranged such finding detectors F in the scan workflow W and they are processed by the scan workflow guidance system 22.

Thus, the shown protocol management system 21 is associated with a suitable scan protocol editor 24 that allows the healthcare provider's central protocol configurators to conveniently edit, configure and manage the large set of scan protocols P. The latter could be setup (e.g., similarly to the Siemens Healthineers MR Dot Cockpit) to allow the protocol configurator to compose scan programs from preconfigured modules. The configurator may assemble the scan programs Pr choosing from a set of, e.g., suitably preconfigured scan protocols, automated planning features and scan workflow guidance features. One preferred variant of the scan protocol editor would allow the customer to compose their scan protocols P from a toolbox T of fundamental building blocks (e.g., sequences, reconstructions, and appropriate parameter sets). The healthcare provider may use the scan protocol editor 24 and the scan program editor 25 to preconfigure their own standardized smart modules S comprised of preconfigured scan protocols P, automated planning and quality assurance steps.

In the scan program editor 25, the set of smart modules S as well as more fundamental elements inside the toolboxes T can be employed to assemble the scan program Pr in a subsequent step. On the right side of Figure 1 an example is shown how a scan program standard can be configured from building blocks inside the various toolboxes T on the left side of Figure 4. In the scan program editor 25, the healthcare provider can define up to n initial smart modules S (e.g., preconfigured protocols P with auxiliary smart components (e.g., automated slice planning, quality assurance steps) for each scan step) from the respective scan workflow toolboxes on the left side in Figure 4. This allows the healthcare provider to setup their scan protocol P standards and derived scan programs Pr in a modular fashion for each clinical indication.

Optionally, a set of finding detectors F can be added after the n-th initial scan protocol P has finished. The finding detectors F will check the acquired image for the appearance of specific features (i.e., findings) and add, replace or remove preconfigured scan steps to/in/from the scan queue 23 based on the type of identified findings. The scan protocol editor 24 and the scan program editor 25 allows the healthcare provider to define which protocols P or smart modules S should be added, replaced, or removed to/in/from the scan queue manager 26 (see figure 5) in case the finding detectors F make a specific observation. Additional meta-information (e.g., priority of the added scan module S) may be configurable for each element for consideration by the scan queue manager 26. The finding detectors F may also take additional input into account (e.g., input from temperature, ECG, pulse, or motion, blood pressure or other vital sign sensors), or other patient information (e.g., lab data, prior images, clinical data) to improve decisions on the workflow W.

One preferred option of the Scan Protocol & Program manager (the scan protocol editor 24 and the scan program editor 25) would include the capability to transfer scan protocols P and scan programs Pr from one scanner configuration to another (e.g., from a 3 T scanner to a 1.5 T scanner).

Figure 5 shows an example of a system 12 according to an embodiment of the invention, comprising a scan-user-interface 20 ("Scan UI input fields"), a scan queue 23, a protocol management system 21 (see also figure 4) and a scan workflow guidance system 22.

The scan workflow guidance system 22 uses the preconfigured scan protocols P and scan programs PR provided by the protocol management system 21 and is here designed to consider subsets of several input types, such as:
a) The clinical indication(s) for the MRI exam, suspected findings or pathologies (e.g., as hypothesized by the referring physician or indicated by the radiologists) to be fed into the scan UI automatically (e.g., from the RIS) or manually by the MR scanner user.
b) MR images as they are acquired and reconstructed by the MR scanner during the initial scan steps of the MRI exam. The protocols of the initial scan steps may follow a preset standard or be configurable (within certain boundaries) by the MR scanner user. E.g., for a standard neurological MRI examination, the initial scan protocols could be a T2 FLAIR axial measurement and a DWI axial measurement with a suitable parameter set.
c) Metrics in a database of morphological data (e.g., derived from clinical cohort studies) allowing to differentiate between normal and abnormal patient morphologies. The Scan Workflow Guidance System may either possess access to this database or exhibit a built-in, local copy of this database.
d) Sensor data acquired during the scan examination (e.g., ECG, breathing, motion detectors).

The shown scan workflow guidance system 22 exhibits a modular architecture of mutually independent analysis branches 27. Each analysis branch 27 comprises an image analysis module 28 (each comprised of, e.g., one or a combination of several deep convolutional neural nets or machine learning based pattern detection algorithms) able to detect one or a specific combination of relevant features from the input MR image and can combine this with other features.

The image analysis modules 28 are special finding detectors F, that find patient-specific circumstances that require a scan workflow adaptation. It should be noted that finding detectors F in general could operate on physiological data (like ECG data) or on images. If they operate on images, they would be realized by the image analysis modules 28 as shown here. Fully analogously, if the finding detector F is configured to operate on physiological data, it could be designated as a (single- or multi-channel) time-trace analyzer module. This, however, does not mean that all image analysis modules 28 always have to be finding detectors F. They could in general also perform other tasks.

The set of relevant feature detectors (the finding detectors associated with the set of image analysis modules 28) may be suitably defined as a set of different detectors for different classes of, e.g., radiological findings, pathologies, image artifacts, or diseases. In the following we provide example sets of detector classes for the application of neurological MR scan workflows W:
Example 1: radiological finding classes: hemorrhage, ischemia, lesion, white matter hyperintensities, degenerative structures, unknown anomalies or pathologies, morphological anomalies.
Example 2: classes of frequent, incidental findings: Cysts, structural vascular abnormality, other developmental variations, infarctions, white matter hyperintensities, micro-hemorrhages, parenchymal calcification, tumors, other pathologies.
Example 3: classes of frequent pathologies: hemorrhage, lesion, MS, dementia, unspecific anomalies.
Example 4: artifact classes: motion artifacts, implant artifacts, aliasing artifact etc.

In case the image analysis module 28 in each analysis branch 27 detects a positive case it may (either directly or following further dissemination/characterization of the detected feature) trigger the output, replacement or deletion of one or a set of preconfigured scan protocols P (incl. appropriate parameters) to/in/from the scan queue manager 26. Ideally, each feature detector is equipped with confidence metric to determine how certain the feature has been detected). In order to properly determine the confidence of the prediction auxiliary analysis modules might be helpful or required (e.g., image quality detection modules).

Alternatively, all or only certain user groups (exhibiting advanced user rights) may be allowed to manually trigger any or all of the finding detectors F by selecting the respective findings in the scan-user-interface 20 (see e.g., figure 3), such that the corresponding scan protocols P will be forwarded to the scan queue manager 26.

The added scan protocols P may be tagged with priority labels according to the prior assessment of the healthcare provider as setup in the scan protocol management system 21.

As indicated above, an important module of the scan workflow guidance system 22 is the scan queue manager 26. It has the central task to curate the set of scan workflow W adaptation as output from the analysis branches 27. Among these tasks we see:
- to curate the set of added protocols (e.g., removing protocol duplicates, merging similar protocols),
- performing consistency checks to ascertain overall scan workflow consistency (e.g., ascertaining that pre and post contrast measurement steps are properly sorted in time with respect to contrast injection or newly introduced parameter settings are consistent across different protocol steps),
- ensuring given time constraints on the available scan work-flow slot (e.g., by considering priority levels of added protocol steps and ascertaining proper coverage of scan workflow elements as predefined in the scan protocol management system 21) .

After each scan session, preferably a report is created by the scan workflow guidance system 22. These reports provide information regarding the initially selected workflow steps, provided suggestions incl. the basis for these suggestions and executed protocol steps incl. parameter settings as well as the executed protocol steps incl. parameter settings, entered comments, and scan times. These reports could also include DICOM objects which might need to be appropriately anonymized/de-identified according to the locally applicable data privacy law.

The bottom left box in figure 2 shows the content of the scan queue 23. The dynamic change of the subsequent protocols happens in the box "Wait for SWF proposals" and can (for regulatory reasons) be confirmed by a user (following box). Alternatively, if the healthcare provider prefers automated execution and no regulatory constrains exist, the subsequent protocols P provided by the scan workflow guidance system 22 could be executed in an automated fashion.

Thus, a master user can configure the available indication-specific scan programs in the scan protocol editor and scan program editor at configuration time to be available in the protocol management system. At scan time the scanner user selects the patient's indication in the UI and thus selects the program to be scanned. This scan program will either be pulled from the protocol management system or exist on the scanner as a local copy until the next update from the master user. Additional, manual adding of protocols can be optionally available, but will not be essential for this invention.

In principle there could be another purple diamond added to the "Scan UI input fields" below with the content "Add all standard neuro protocols?" which could be named "Manually add protocols?" which could have a direct connection to the protocol management system. Since the invention primarily focusses on the automatic adaption, this diamond and line was not added to remove complexity.

Although the present invention has been disclosed in the form of preferred embodiments and variations thereon, it will be understood that numerous additional modifications and variations could be made thereto without departing from the scope of the invention. For the sake of clarity, it is to be understood that the use of "a" or "an" throughout this application does not exclude a plurality, and "comprising" does not exclude other steps or elements. The mention of a "unit" or a "device" does not preclude the use of more than one unit or device.

## Claims

1. A system (12) for standardized MRI examinations with patient-centric scan workflow (W) adaptations comprising:
- a protocol management system (21) for setup and/or to maintain scan programs (Pr) and/or scan protocols (P, PI) centrally,
- a scan queue (23) designed to provide a representation of past and planned scan workflow steps,
- a scan workflow guidance system (22) which is configured to use a preconfigured scan protocol (P, PI) and/or scan program (Pr) by patient specific adaptions.

2. The system according to claim 1, wherein the protocol management system (21) comprises a centralized database for storing scan protocols (P, PI), preferably wherein the protocol management system (21) comprises a default set of scan protocols (P, PI) and/or scan programs (Pr) stored in a centralized, operation-wide accessible database (D), preferably with tiered access control, wherein the set of scan protocols (P, PI) and/or scan programs (Pr) is linked to a protocol identifier (Pi), especially with the information selected from the group sequence with preconfigured parameter sets, reconstruction with preconfigured parameter sets, automated quality assurance steps, post-processing operations to be performed on acquired images and other relevant parameters.

3. The system according to one of the preceding claims, wherein the protocol management system (21) comprises a scan protocol editor (24) and/or a scan program editor (25), each with different building blocks, wherein a scan protocol (P, PI) can preferably be contained in different modules of the different building blocks, preferably wherein
- the scan protocol editor (24) is designed to edit, configure, and manage the set of scan protocols (P, PI) comprised by the protocol management system (21), especially designed for composing scan protocols (P, PI) from a toolbox of fundamental building blocks,
- the scan program editor (25) is designed to assemble a scan program (Pr) with a set of smart modules (S) comprised by toolboxes.

4. The system according to one of the preceding claims, wherein the scan workflow guidance system (22) is configured to consider all or any subsets of the following four input types:
a) the clinical indication(s) for the MRI exam, suspected findings or pathologies to be fed into a scan-user-interface (20) automatically or manually by the MR scanner user,
b) MR images as they are acquired and reconstructed by the MR scanner during the initial scan steps of the MRI exam. The protocols of the initial scan steps may follow a preset standard or be configurable by the MR scanner user, the initial scan protocols (P) could be a T2 FLAIR axial measurement and a DWI axial measurement with a suitable parameter set,
c) metrics in a database of morphological data allowing to differentiate between normal and abnormal patient morphologies,
d) sensor data acquired during the scan examination.

5. The system according to one of the preceding claims, comprising a scan-user-interface (20), which is configured to modify a scan workflow (W) by a user, preferably wherein the scan-user-interface (20) is configured to review and/or edit the scan workflow (W), especially to insert the scan indication or suspected pathologies of the patient, preferably in a tiered privilege level concept,
preferably wherein the scan-user-interface (20) allows the user to add a set of standard scan protocols (P) of the respective application, regardless of the recommendations determined by the scan workflow guidance system (22) downstream, especially wherein the scan-user-interface (20) is equipped with an option to review and edit scan workflow (W) adaptation as suggested by the scan workflow guidance system (22) .

6. The system according to one of the preceding claims, comprising a scan queue (23) designed to provide a representation of the past and planned scan workflow steps at any given time, preferably wherein planned scan workflow steps may be subject to change through the scan workflow guidance system (22) and user interaction.

7. A method for standardized MRI examinations with patient-centric scan workflow (W) adaptations performed with a system (12) according to any of the preceding claims, comprising the steps:
- provide a scan protocol to a scan workflow (W) in the scan queue (23),
- performing a number of scans according to the scan workflow (W),
- performing patient-specific adaptions of the scan workflow (W) by the scan workflow guidance system (22) with preconfigured scan protocols (P, PI) and scan programs (Pr), based on considerations pertaining to:
a) clinical indication(s) for the MRI exam, suspected findings or pathologies to be fed into the scan-user-interface (20) automatically or manually by an MR scanner user,
b) MR images as they are acquired and reconstructed by the MR scanner during the initial scan steps of the MRI exam,
c) metrics in a database of morphological data allowing to differentiate between normal and abnormal patient morphologies,
d) sensor data acquired during the scan examination,
- performing a number of scans according to the adapted scan workflow (W).

8. The method according to claim 7, wherein a set of finding detectors (F) is added after the n-th initial scan protocol (P) has finished, the finding detectors (F) checking the acquired image for the appearance of specific features and adding, replacing, or removing preconfigured scan steps to/in/from a scan queue (23) based on the type of identified findings, preferably wherein a scan protocol editor (24) and/or a scan program editor (25) allow a healthcare provider to define which scan protocols (PI) or smart modules (S) should be added, replaced or removed to/in/from a scan queue manager (26) in case the finding detectors (F) make a specific observation.

9. The method according to claim 7 or 8, wherein the scan workflow guidance system (22) reports a suggested and/or executed scan workflow (W) to be archived and accessible in the protocol management system (21), preferably wherein these reports can be accessed via an analysis tool providing insights into scan protocol (P, P1) usage, circumstances under which the suggestions of the scan workflow guidance system (22) where followed vs. not followed, and a large set of statistical data derived from the scanner's scan history across the institutions scanner fleet,
preferably wherein a user is able to confirm or change the scan workflow (W) recommendations of the scan workflow guidance system (22) before execution.

10. A scan workflow guidance system (22) adapted for a system (12) according to any of claims 1 to 6, configured to use a preconfigured scan protocol (P, P1) and/or scan program (Pr) with the capability to perform patient specific adaptions, wherein the scan protocols (P) and/or scan programs (Pr), which are selected by a user, are getting adapted based on acquired data and/or user selected presetting.

11. The scan workflow guidance system according to claim 10, comprising a modular architecture of mutually independent analysis branches (27), wherein each analysis branch (27) comprises an image analysis module (28), especially comprised of one or a combination of several deep convolutional neural nets or machine learning based pattern detection algorithms, able to detect one or a specific combination of relevant features from an input MR image and able to combine these with other features,
preferably wherein the scan workflow guidance system (22) comprises a set of relevant feature detectors (F) associated with the set of image analysis modules (28) that is especially suitably defined as a set of different detectors for different classes of radiological findings, pathologies, image artifacts, or diseases.

12. The scan workflow guidance system according to claim 11, comprising a scan queue manager designed to curate a set of scan workflow adaptation as output from the analysis branches, wherein the scan queue manager is preferably designed to achieve one or more of the tasks:
- curating the set of added protocols,
- performing consistency checks to ascertain overall scan workflow consistency,
- ensuring given time constraints on the available scan workflow slot.

13. a magnetic imaging system comprising a system according to any of claims 1 to 6 and preferably to perform a method according to any of claims 7 to 9.

14. A computer program product comprising a computer program that is directly loadable into a system (12) or a control device (13) for a magnetic resonance imaging system (1), which comprises program elements for performing steps of the method according to any of claims 7 to 9 when the computer program is executed by the system (12) or the control device (13).

15. A computer-readable medium on which is stored program elements that can be read and executed by a computer unit in order to perform steps of the method according to any of claims 7 to 9 when the program elements are executed by the computer unit.
